(19) Europäisches Patentamt
European Patent Office
Office européen des brevets

(11) Numéro de publication: **0 073 718**
**A1**

(12) **DEMANDE DE BREVET EUROPEEN**

(21) Numéro de dépôt: **82401585.3**

(22) Date de dépôt: **26.08.82**

(51) Int. Cl.[3]: **B 01 J 21/16**, C 01 B 33/26, B 01 J 29/02

(30) Priorité: **27.08.81 FR 8116387**

(43) Date de publication de la demande: **09.03.83**
**Bulletin 83/10**

(84) Etats contractants désignés: **BE DE FR GB IT NL**

(71) Demandeur: **Jacobs, Pierre, 6 Populierenstraat, B-1686 Gooik (BE)**
Demandeur: **Poncelet, Georges, 15 rue de Wavre, B-5998 Beauvechain (BE)**
Demandeur: **Schutz, Alain, 4 rue Godincourt, B-6778 Musson (BE)**

(72) Inventeur: **Jacobs, Pierre, 6 Populierenstraat, B-1686 Gooik (BE)**
Inventeur: **Poncelet, Georges, 15 rue de Wavre, B-5998 Beauvechain (BE)**
Inventeur: **Schutz, Alain, 4 rue Godincourt, B-6778 Musson (BE)**

(74) Mandataire: **Jolly, Jean-Pierre et al, Cabinet BROT et JOLLY 83, rue d'Amsterdam, F-75008 Paris (FR)**

(54) **Procédé de préparation d'argiles pontées, argiles préparées par ce procédé et applications desdites argiles.**

(57) L'invention concerne un procédé de préparation d'argiles pontées.

Selon l'invention, on effectue la dialyse d'un mélange d'une solution aqueuse d'au moins un hydroxyde d'un métal et d'une suspension aqueuse d'argile.

Ces argiles peuvent servir de catalyseurs ou de supports de catalyseurs pour la conversion d'au moins un hydroxyde d'un métal et d'une suspension aqueuse d'argile.

Ces argiles peuvent servir de catalyseurs ou de supports de catalyseurs pour la conversion d'hydrocarbures paraffiniques ou oléofiniques.

EP 0 073 718 A1

Procédé de préparation d'argiles pontées, argiles préparées par ce procédé et applications desdites argiles.

La présente invention concerne un procédé de préparation d'argiles pontées, les argiles préparées par ce procédé et des applications desdites argiles.

Certaines argiles ont une structure à réseau expansible. Elles ont la propriété de pouvoir adsorber, notamment, de l'eau entre les différents feuillets qui les composent. C'est le cas, en particulier, des argiles du groupe de la montmorillonite. Ces argiles ont une structure que l'on peut définir, d'une manière simplifiée, comme une structure composée de feuillets à couches triples comportant deux couches simples de tétraèdres $Si\ O_4$ et une couche intermédiaire dioctaédrique ou trioctaédrique. La cohésion entre les couches est assurée par la mise en commun d'atomes d'oxygène apicaux. Les atomes de silicium des tétraèdres peuvent être remplacés en partie par des atomes d'aluminium.

De la même façon, dans les octaèdres de la couche intermédiaire $AlX_6$ (X = O, OH), les atomes d'aluminium peuvent être remplacés en partie par des atomes de magnésium ou de fer. On connaît les montmorillonites ferrifères, appelées nontronites, dans lesquelles la couche octaédrique est entièrement constituée d'octaèdres de fer, la charge du feuillet étant issue de substitutions au niveau des couches tétraédriques. La saponite, qui est une argile trioctaédrique (couche octaédrique magnésienne), possède une charge provenant de substitutions au niveau des couches tétraédriques siliciques.

Il est bien connu que la surface spécifique d'un support a une très grande importance en catalyse et ces argiles, du fait de leur propriété d'être expansibles, pourraient être utilisées comme catalyseurs ou supports de catalyseurs, notamment pour la conversion d'hydrocarbures. Mais, une fois expansées, c'est-à-dire après adsorption de l'eau entre leurs différents feuillets, ces argiles ont l'inconvénient de perdre leur caractère expansé par chauffage à 100°C et, de ce fait, de ne pas

conserver l'augmentation de surface spécifique pouvant résulter de leur expansion.

On peut rappeler que l'état d'expansion d'une argile est défini par l'espacement interfoliaire et l'espacement basal, qui sont mesurés par diffraction de rayons X.

L'espacement interfoliaire est, comme son nom l'indique, l'espacement entre deux feuillets. A l'état non expansé, après séchage de l'argile, il est donc nul.

L'espacement basal, représenté par le sigle $d_{001}$, est défini comme la somme de l'épaisseur d'un feuillet et de l'espacement interfoliaire.

Dans le cas de la montmorillonite, l'épaisseur d'un feuillet est de 9,6 angstrõms. A l'état expansé, l'espacement interfoliaire peut dépasser environ 10 angstrõms et l'espacement basal peut donc dépasser environ 20 angströms.

On a cherché, dans le but notamment d'utiliser les argiles expansibles comme supports de catalyseurs ou comme catalyseurs, à obtenir des argiles ayant un espacement basal aussi élevé que possible, cet espacement basal pouvant être maintenu quand l'argile est soumise à un traitement thermique.

On a ainsi réussi à introduire entre les feuillets d'argile des piliers ou ponts pour obtenir des argiles qui seront désignées, dans la suite de la présente description, par le terme "argiles pontées".

Une méthode bien connue consiste à introduire, entre les feuillets d'argile, des ponts constitués par des oligomères d'un hydroxyde d'un métal et, notamment, d'un hydroxyde d'aluminium.

Cette méthode, notamment décrite dans l'article de MM. LAHAV, U.SHANI et J. SHABTAI, paru dans Clays and Clays Mineral, Volume 26, numéro 2, pages 107 à 115 (1978), consiste à mettre en contact un oligomère d'hydroxyde d'aluminium avec une suspension aqueuse de montmorillonite. La méthode décrite permet d'obtenir un espacement basal d'environ 18 angströms. Cet article indique qu'il est

nécessaire de faire vieillir la solution hydroxy-aluminique obtenue par réaction de la soude sur du chlorure d'aluminium, avant de la mettre en contact avec la suspension d'argile. Ce processus est donc relativement long. En outre, les suspensions d'argile employées sont très peu concentrées, 50 à 200 mg par litre de suspension, soit donc 0,005 % à 0,02 % en poids. Cette méthode nécessite en conséquence la manipulation de grands volumes de suspension, ce qui est naturellement un inconvénient.

De plus, l'article indique qu'il est préférable d'opérer avec un rapport $\frac{OH^-}{Al^{3+}}$ relativement élevé (2,33). Or, quand on opère avec un rapport $\frac{OH^-}{Al^{3+}}$ voisin de 3, on risque une cristallisation incontrôlée de bayérite et/ou de gibbsite $Al\ (OH)_3$ en dehors des feuillets, ce qui est un inconvénient supplémentaire.

La méthode décrite dans cet article présente donc plusieurs inconvénients.

Une autre méthode décrite dans le brevet français n°2 394 324 consiste à mettre en contact l'argile avec une solution de chlorhydroxyde d'aluminium appelé "chlorhydrol". Cette méthode présente également des inconvénients, car la mise en contact de l'argile et du "chlorhydrol" doit être effectuée à une température relativement élevée (dans les exemples du brevet indiqué ci-dessus, on cite des températures de l'ordre de 70°C) et nécessite aussi un vieillissement.

L'invention vise à proposer une nouvelle méthode de préparation d' "argiles pontées" ne présentant pas les inconvénients de la technique connue énumérés ci-dessus.

A cet effet, l'invention a pour objet un procédé de préparation d'argiles pontées, caractérisé en ce que l'on effectue la dialyse d'un mélange d'une solution aqueuse d'au moins un hydroxyde d'un métal et d'une suspension aqueuse d'argile.

Par solution aqueuse d'au moins un hydroxyde d'un métal, on entend une solution contenant des ions $OH^-$ et des ions $M^x$ d'au moins un métal dans laquelle le rapport $\frac{OH^-}{M^x}$ est tel que le seuil de précipitation de l'hydroxyde n'est pas atteint.

Selon l'invention, l'hydroxyde peut être choisi dans le groupe formé par les hydroxydes des éléments des groupes IIA, IIIA, IVA, VA, VIA, VIIA, VIII, IB, IIB, IIIB, IVB, VB et VIB de la classification périodique des éléments.

Dans le procédé selon l'invention, l'hydroxyde peut être obtenu par action d'une base sur une solution d'un sel d'un métal dans laquelle le métal se trouve sous forme de cation, ou d'un acide sur une solution d'un sel d'un métal dans laquelle le métal se trouve sous forme d'anion.

Le mélange de la solution d'au moins un hydroxyde d'un métal et de la suspension d'argile peut être réalisé de différentes façons :

- soit en préparant la solution d'hydroxyde, par exemple par action d'une base sur un sel, puis en ajoutant la solution obtenue à la suspension d'argile,
- soit en ajoutant à la suspension d'argile une solution du sel du métal, puis en ajoutant une solution de base,
- soit encore en ajoutant simultanément à la suspension d'argile une solution du sel du métal et une base.

Autrement dit, la solution d'hydroxyde peut être préparée avant son mélange avec la suspension d'argile, ou en ajoutant à la suspension d'argile les précurseurs de la solution d'hydroxyde.

La solution d'hydroxyde peut être également obtenue à partir de certains composés qui s'hydrolysent spontanément, sans que l'on ait besoin de rajouter une base, par exemple à partir des oxychlorures de zirconium, de vanadium, de molybdène ou de rhénium.

Dans le procédé selon l'invention, un des paramètres importants est le rapport des ions $\frac{OH^-}{M^x}$ dans la solution d'hydroxyde, où x définit le degré d'oxydation du métal.

Quand x = I, le rapport $\frac{OH^-}{M^I}$ peut être compris entre 0,2 et 1, de préférence entre 0,2 et 0,8 et, mieux encore, entre 0,3 et 0,7.

Quand x = II, le rapport $\frac{OH^-}{M^{II}}$ peut être compris entre 0,2 et 2, de préférence entre 0,4 et 1,8 et, mieux encore, entre 0,5 et 1,4.

Quand x = III, le rapport $\frac{OH^-}{M^{III}}$ peut être compris entre 0,3 et 3, de préférence entre 0,6 et 2,4 et, mieux encore, entre 0,8 et 1,8.

Quand x = IV, le rapport $\frac{OH^-}{M^{IV}}$ peut être compris entre 0,4 et 4, de préférence entre 0,8 et 3,6 et, mieux encore, entre 1 et 2,8.

Dans le procédé selon l'invention, la concentration, dans le mélange de la suspension d'argile et de la solution d'hydroxyde, de l'ion du métal utilisé pour former les piliers d'oxydes métalliques doit être telle qu'elle soit suffisamment importante pour qu'il y ait formation de piliers d'oxydes métalliques et ne pas être trop élevée pour qu'il n'y ait pas trop d'oxydes métalliques insérés entre les feuillets d'argile, de façon à ne pas nuire à la porosité de l'argile.

Elle peut être comprise par exemple, exprimée en milliéquivalents (m.e.q.) de l'ion du métal considéré par gramme d'argile, entre 6 et 60 et, de préférence, entre 10 et 30.

La concentration en argile dans le mélange final, c'est-à-dire après mélange de la solution d'hydroxyde et de la suspension d'argile initiale, doit être telle qu'elle soit suffisamment importante pour ne pas avoir à manipuler de grands volumes de mélange, et ne pas être

trop élevée, car si le mélange est trop concentré en argile, il est difficile à manipuler.

La concentration en argile du mélange final peut être par exemple comprise entre 0,1 et 4 % en poids et, de préférence, comprise entre 0,8 et 1,5 % en poids.

Les argiles qui peuvent être utilisées dans le procédé selon l'invention, peuvent être choisies dans le groupe constitué par des argiles gonflantes (smectites), naturelles ou obtenues par voie de synthèse hydrothermale.

L'étape finale du procédé selon l'invention consiste à effectuer la dialyse du mélange de la solution d'au moins un hydroxyde d'un métal et de la suspension d'argile.

Cette dialyse peut être effectuée en plaçant ledit mélange dans une enceinte constituée par une membrane semi-perméable choisie dans le groupe constitué par les membranes à base de cellulose régénérée ou toute autre membrane semi-perméable.

L'enceinte est ensuite plongée dans de l'eau, de préférence de l'eau déminéralisée, bien que toute eau (distillée, eau de ville) puisse convenir, du moment qu'elle a une force ionique inférieure à celle de l'eau contenue dans la membrane de dialyse.

Cette opération peut être effectuée en discontinu ou en continu, c'est-à-dire, dans ce dernier cas, en plaçant l'enceinte dans un courant d'eau.

L'opération de dialyse peut être effectuée à la température ambiante, mais peut être activée en élevant la température de l'eau. La température limite est fixée par la température d'ébullition de l'eau à la pression à laquelle l'opération est effectuée.

Les argiles préparées par le procédé selon l'invention ont une très bonne stabilité thermique et conservent leur espacement basal, quand elles sont portées à haute température. Elles peuvent donc servir de supports de catalyseurs et de catalyseurs, notamment dans la conversion d'hydrocarbures.

Elles peuvent, en particulier, servir de catalyseurs ou de supports pour des catalyseurs d'isomérisation d'hydrocarbures paraffiniques ou d'oléfines, ou de craquage

de ces hydrocarbures, éventuellement en présence d'hydrogène.

On peut ainsi disposer sur ces argiles au moins un des éléments des groupes IA, IIA, IIIA, IVA, VA, VIA, VIIA, VIII, IB, IIB, IIIB, IVB, VB et VIIB de la classification périodique des éléments.

L'invention est illustrée à titre non limitatif par les six exemples qui suivent.

Les cinq figures jointes à la présente description seront explicitées dans ces exemples.

EXEMPLE 1

Cet exemple concerne la préparation d'argiles pontées A1, A2, A3, A4, A5, A6, A7, A8, A9, A10, A11, A12, A13 et A14, par le procédé selon l'invention, ainsi que d'argiles pontées témoin TA2, TA4 et TA6, par un procédé voisin, mais ne comportant pas l'opération finale de dialyse.

On part d'une montmorillonnite, que l'on sature par des ions sodium $Na^+$ en la traitant plusieurs fois par une solution aqueuse de chlorure de sodium normale.

On élimine l'excès de chlorure de sodium par centrifugation. On lave plusieurs fois l'argile par de l'eau déminéralisée en effectuant une centrifugation entre chaque lavage.

La fraction dont la granulométrie est inférieure ou égale à 2 microns est séparée par décantation, la fraction dont la granulométrie est supérieure à 2 microns étant éliminée.

On prépare, à partir de cette fraction d'argile, des suspensions aqueuses S1 et S2, contenant respectivement 2,5 % et 4,16 % en poids d'argile.

On prépare également :

- une solution I aqueuse de chlorure d'aluminium $AlCl_3, 6\ H_2O$ ;
- une solution II aqueuse de chlorure de chrome $CrCl_3, 6\ H_2O$ ;

- une solution III aqueuse de soude NaOH.

Les trois solutions I,II et III ont une concentration molaire de 0,2.

On ajoute la solution III à la solution I et/ou la solution II en quantités variables, lentement, sous agitation continue, de manière à éviter la formation ou apparition d'un trouble. On obtient ainsi différentes solutions d'hydroxydes dont le rapport $\frac{OH^-}{Al^{3+}}$ ou $\frac{OH^-}{Cr^{3+}}$ ou $\frac{OH^-}{Cr^{3+} + Al^{3+}}$ varie.

Les différentes solutions d'hydroxyde obtenues sont mélangées sous agitation en quantités variables à une certaine quantité de la suspension S1 ou S2.

On obtient ainsi des mélanges contenant des concentrations variables de métal (ou de métaux).

Pour préparer les argiles témoin TA2, TA4 et TA6, on lave simplement certains des mélanges obtenus avec de l'eau déminéralisée.

Pour préparer des argiles par le procédé selon l'invention, les mélanges obtenus sont placés dans une poche composée d'une membrane cellulosique semi-perméable, de porosité moyenne de 24 Angströms, commercialisée sous la marque VISKING par la Société MEDICELL International Ltd.

La poche est placée dans 10 litres d'eau déminéralisée pendant 24 heures. L'opération est renouvelée quatre fois.

Après cette opération de dialyse, on prélève sur les produits obtenus par les deux procédés des échantillons, qui sont examinés par diffraction aux rayons X.

On centrifuge les suspensions et les différents produits obtenus sont séchés par lyophilisation.

Les conditions de préparation des argiles pontées obtenues et leur espacement basal mesuré par diffraction de rayons X sont donnés dans le Tableau I ci-après.

TABLEAU I

| Argile préparée | Quantités des solutions ou suspensions engagées pour la préparation de l'argile (en millilitres) | | | | | Rapport des ions $\frac{OH^-}{M^{3+}}$ (6) | Concentration en m.e.q. par gramme d'argile (7) | % molaire $\frac{Cr^{3+}}{Cr^{3+}+Al^{3+}}$ (8) | Espacement basal (9) |
|---|---|---|---|---|---|---|---|---|---|
| | Solution I (1) | Solution II (2) | Solution III (3) | Suspension d'argile $S_1$ (4) | Suspension d'argile $S_2$ (5) | | | | |
| A1 | 333 | 0 | 266 | 400 | 0 | 0,8 | 20 | - | 19 |
| A2 | 333 | 0 | 333 | 400 | 0 | 1,0 | 20 | - | 19 |
| TA2 | 333 | 0 | 333 | 400 | 0 | 1,0 | 20 | - | 16,2 |
| A3 | 333 | 0 | 400 | 400 | 0 | 1,2 | 20 | - | 19 |
| TA3 | 333 | 0 | 400 | 400 | 0 | 1,2 | 20 | - | 16,2 |
| A4 | 333 | 0 | 466 | 400 | 0 | 1,4 | 20 | - | 18,2 |
| TA4 | 333 | 0 | 466 | 400 | 0 | 1,4 | 20 | - | 17,3 |
| A5 | 333 | 0 | 533 | 400 | 0 | 1,6 | 20 | - | 19,1 |
| A6 | 333 | 0 | 566 | 400 | 0 | 1,7 | 20 | - | 18 |
| TA6 | 333 | 0 | 566 | 400 | 0 | 1,7 | 20 | - | 12,4 |
| A7 | 333 | 0 | 666 | 400 | 0 | 2,0 | 20 | - | 19,5 |
| A8 | 166,5 | C | 333 | 0 | 140 | 2,0 | 10 | - | 18 |

TABLEAU I (suite)

| Argile préparée | Quantités des solutions ou suspensions engagées pour la préparation de l'argile (en millilitres) | | | | | Rapport des ions $\frac{OH^-}{M^{3+}}$ (6) | Concentration en m.e.q. par gramme d'argile (7) | % molaire $\frac{Cr^{3+}}{Cr^{3+}+Al^{3+}}$ (8) | Espacement basal (9) |
|---|---|---|---|---|---|---|---|---|---|
| | Solution I (1) | Solution II (2) | Solution III (3) | Suspension d'argile $S_1$ (4) | Suspension d'argile $S_2$ (5) | | | | |
| A9 | 166,5 | 0 | 383 | 0 | 240 | 2,3 | 10 | - | 17,9 |
| A10 | 166,5 | 0 | 416 | 0 | 240 | 2,5 | 10 | - | 17,9 |
| A11 | 0 | 333 | 466 | 400 | 0 | 1,4 | 20 | - | 17,6 |
| A12 | 0 | 333 | 666 | 400 | 0 | 2,0 | 20 | - | 19 |
| A13 | 230 | 100 | 333 | 400 | 0 | 1,0 | 20 | 30 | 17,6 |
| A14 | 165 | 165 | 333 | 400 | 0 | 1,0 | 20 | 50 | 17,2 |

(1) Solution d'Al $Cl_3$, $6H_2O$ 0,2 molaire.

(2) Solution de Cr $Cl_3$, $6H_2O$ 0,2 molaire.

(3) Solution de NaOH 0,2 molaire.

(4) Suspension à 2,5% en poids.

(5) Suspension à 4,16% en poids.

(6) Dans la solution d'hydroxyde du métal ou des métaux (dans le cas d'une solution mixte d'Al et de Cr).

(7) Concentration de l'ion ou des ions des métaux (dans le cas d'une solution mixte d'Al et de Cr) dans le mélange solution d'hydroxyde + suspension d'argile.

(8) Dans le cas d'une solution mixte d'Al et de Cr.

(9) Après séchage à la température ambiante.

On peut constater, d'après ce tableau, que l'espacement basal des argiles préparées par le procédé selon l'invention est plus élevé que celui des argiles préparées par un procédé sans dialyse. On peut, en particulier, comparer les argiles A6 et TA6.

Le spectre de diffraction des rayons X des argiles A6 et TA6 séchées à température ambiante est donné sur la figure 1 annexée.

On constate que l'espacement basal de l'argile A6 est plus élevé que celui de l'argile TA6, et que la raie de l'argile A6 est beaucoup plus étroite et intense que celle de l'argile TA6. Ceci indique que la couche hydroxy aluminique intercalée entre les feuillets est beaucoup mieux organisée dans l'argile A6 que dans l'argile TA6.

On soumet ensuite les argiles ainsi préparées à différents traitements thermiques. On mesure après chaque traitement leur espace basal. Les résultats avant et après traitement thermique figurent dans le Tableau II ci-après.

TABLEAU II

| Argile | Avant traitement thermique | Espacement basal en Angströms | | | | | |
|---|---|---|---|---|---|---|---|
| | | Conditions du traitement thermique | | | | | |
| | | 2 heures à 100 °C | 2 heures à 200 °C | 1 heure à 300 °C | 1 heure à 400 °C | 1 heure à 500 °C | 1 heure à 550 °C |
| A1 | 19 | | | 18,1 | | 17,1 | |
| A2 | 19 | | | 18,1 | | 17,1 | |
| TA2 | 16,2 | | | 11,2 | | 9,7 | |
| A3 | 19 | | | 18 | | 17 | |
| TA3 | 16,2 | | | 11,2 | | 9,4 | |
| A4 | 18,2 | | 17,8 | | 17,3 | | 16,9 |
| TA4 | 17,3 | 17 | 12,3 | | 9,7 | | 9,7 |
| A5 | 19,1 | | | 17,6 | | 16,3 | |
| A6 | 18 | | | | | | |
| TA6 | 12,4 | | | | | | |
| A7 | 19,5 | | | 17 | | 16 | |
| A8 | 18 | | | 17,3 | | 16,9 | |
| A9 | 17,9 | | | 17,3 | | 16,8 | |
| A10 | 17,9 | | | 17,2 | | 16,3 | |
| A11 | 17,6 | | | 16,4 | | | |
| A12 | 19 | 18 | | 17 | | | |
| A13 | 17,6 | | | 14,7 | | | |
| A14 | 17,2 | | | 14,5 | | | |

Le tableau II permet de constater que l'espacement basal des argiles préparées par le procédé selon l'invention ne diminue que légèrement avec la température, ce qui est un avantage, naturellement, dans le cas de l'emploi de ces argiles comme supports de catalyseurs ou comme catalyseurs.

Il n'en est pas de même pour les argiles témoin préparées par un procédé sans dialyse.

On a représenté sur les figures 2a et 2b, à titre d'exemple, les pics obtenus par diffraction de rayons X pour les argiles A4 et TA4 respectivement, qui montrent bien que l'espacement basal de l'argile TA4 diminue avec le traitement thermique.

On a mesuré les surfaces spécifiques des différentes argiles pontées, à partir des isothermes d'adsorption d'azote établis à la température de l'azote liquide, selon la méthode B.E.T. Les surfaces spécifiques sont comprises entre 200 et 400 $m^2$/gramme. Les argiles sont préalablement dégazées sous vide à 300°C, pendant 16 heures.

La distribution de la porosité établie à partir des isothermes de désorption d'azote est binodale, avec des pores de diamètre de l'ordre de 40 à 50 Angströms et des pores de diamètre compris entre 10 et 20 Angströms.

EXEMPLE 2

Cet exemple concerne la préparation d'une argile pontée B par le procédé selon l'invention.

On part d'une montmorillonite qui est traitée de la même façon que dans l'exemple 1, c'est-à-dire échange à l'aide de chlorure de sodium puis séparation de la fraction, dont la granulométrie est inférieure ou égale à 2 microns.

A une suspension aqueuse de cette montmorillonite contenant 2,5 % en poids de cette argile, on ajoute 30 millilitres d'une solution, 0,2 molaire d'oxychlorure de zinc $ZnOCl_2$. On ajoute 130 millilitres d'eau distillée, de façon à obtenir une suspension aqueuse contenant 0,5 % en poids d'argile.

La suspension est dialysée à l'aide d'une membrane identique à celle utilisée dans l'exemple 1 à raison de 1 litre d'eau déminéralisé par gramme d'argile.

L'eau de dialyse est renouvelée après 24 heures. On prélève un échantillon après chaque dialyse afin d'examen par diffraction aux rayons X.

Les échantillons sont séchés à température ambiante, puis soumis à différents traitements thermiques.

Les résultats, avant et après traitement thermique, figurent dans le tableau III ci-après :

Tableau III

| Traitement | Espacement basal en Angstrõms | | | |
|---|---|---|---|---|
| | Après une dialyse | Après trois dialyses | Après qatre dialyses | Après cinq dialyses |
| Séchage à température ambiante | 11,3 | 19,6 | 18,5 | 19 |
| à 110°C | 11 | 16,4 | 16,1 | 16,4 |
| à 400°C | 10 | 15,7 | 15,7 | 15,7 |

L'influence de la dialyse ressort clairement de ce tableau. L'espacement basal reste assez élevé malgré le traitement thermique.

EXEMPLE 3

Cet exemple concerne la préparation d'une argile pontée C par le procédé selon l'invention et d'une argile pontée témoin TC par un procédé ne comportant pas l'opération finale de dialyse.

On part d'une fraction de montmorillonite identique à celle des exemples 1 et 2.

A une suspension aqueuse de cette montmorillonite, on ajoute du sulfate de titane Ti $(SO4)_2$ en solution acide à raison de 33 mg de titane par gramme d'argile.

La concentration finale de la suspension d'argile est de 0,25 % en poids. Un échantillon est prélevé et séché.

Il s'agit de l'argile témoin TC.

La suspension est dialysée de la même façon que dans l'exemple 2 et des échantillons sont prélevés, traités et examinés de la même façon.

Les résultats figurent dans le tableau IV ci-après :

TABLEAU IV

| Traitement | Espacement basal en Angströms | | | |
|---|---|---|---|---|
| | Argile témoin TC (Sans dialyse) | Argile C après | | |
| | | une dialyse | deux dialyses | trois dyalises |
| Séchage à température ambiante | 12,2 | 12,4 | 23,2 | 23 |
| à 110°C | non mesuré | 12,1 | 20 | 23,2 |
| à 300°C | non mesuré | non mesuré | non mesuré | 23,8 |

L'influence de la dialyse ressort clairement de ce tableau. L'espacement basal reste très élevé malgré le traitement thermique.

Exemple 4

Cet exemple concerne la préparation d'une argile pontée D par le procédé selon l'invention et d'une argile pontée témoin TD par un procédé ne comportant pas l'étape finale de dialyse.

On part d'une fraction de montmorillonite identique à celle des exemples 1, 2 et 3.

A une suspension aqueuse de cette montmorillonite, contenant 2,5 % en poids d'argile, on ajoute 20 meq de holmium par g d'argile sous forme de nitrate de holmium $Ho(NO_3)_3$, $6H_2O$ en solution aqueuse.

La suspension aqueuse finale contient 1 % en poids d'argile.

La suspension est dialysée de la même façon que dans l'exemple 3 et des échantillons sont prélevés, traités et examinés de la même façon.

Le diffractogramme de rayons X montre que la raie 001 s'affine considérablement en fonction du nombre de dialyses et augmente en intensité, ce qui traduit l'effet bénéfique de la dialyse sur l'organisation régulière des feuillets, elle-même conditionnée par l'agencement des piliers.

Sur le tableau V ci-après sont représentées les intensités de la raie 001 à 15,5 A°, en fonction du nombre de dialyses.

Les intensités sont mesurées sur le diffractogramme de rayons X et multipliées par le nombre de coups par seconde.

TABLEAU V

| Traitement | Intensité de la raie 001 multipliée par le nombre de coups/seconde | | | |
|---|---|---|---|---|
| | Argile témoin TD (sans dialyse) | Argile D après | | |
| | | une dialyse | deux dialyses | trois dialyses |
| Séchage à température ambiante | 1 220 | 7 500 | 11 000 | 10 000 |
| à 110°C | 680 | 2 680 | 2 800 | non mesuré |
| à 300°C | 120 | 1 120 | 1 640 | 3 000 |

L'influence de la dialyse ressort clairement de ce tableau et demeure malgré le traitement thermique.

EXEMPLE 5

Cet exemple concerne l'hydroisomérisation et l'hydrocraquage d'une charge de normal-décane à l'aide de catalyseurs préparés à partir des argiles de l'exemple 1.

BAD ORIGINAL

On prépare, à partir de différentes argiles de l'exemple 1, des catalyseurs contenant 1 % en poids de platine, en imprégnant les argiles à l'aide d'une solution aqueuse de chlorure de platine tétramine, Pt $(NH_3)_4$ $Cl_2$.

Après séchage à 110°C, pendant 16 heures, on calcine à l'air les produits obtenus, puis on les réduit par un courant d'hydrogène.

On obtient des catalyseurs dont les références figurent dans le tableau VI ci-après :

TABLEAU VI

| Argile de départ | Catalyseur |
|---|---|
| A2 | C2 |
| A3 | C3 |
| A4 | C4 |
| A7 | C7 |
| A8 | C8 |
| A9 | C9 |
| A10 | C10 |
| A11 | C11 |
| A13 | C13 |
| A14 | C14 |

On effectue ensuite, pour chaque catalyseur, un essai d'hydroisomérisation et d'hydrocraquage de normal décane, en plaçant une quantité donnée de catalyseur dans un réacteur de 5 millimètres. On fait alors passer sur le catalyseur un mélange de normal-décane et d'hydrogène à la pression atmosphérique, avec un débit $F_o$ de normal-décane en tête du réacteur tel que, W étant le poids de catalyseur, le rapport $\frac{W}{F_o}$ soit égal à 518 kg.seconde/mole, avec

un rapport hydrogène égal à 71 normaux litre/litre.
normal-décane

Les essais sont conduits en élevant la température de 100°C à 300°C pour avoir la conversion totale de normal-décane.

On repère successivement, en analysant par chromatographie en phase gazeuse les gaz sortant du réacteur :

- la température à laquelle il y a 20 % de conversion de normal-décane,

- la température à laquelle il y a le maximum d'isomérisation,

- la température à laquelle il y 20 % de craquage.

Les quantités de catalyseur utilisées et les résultats des essais figurent dans le Tableau VII ci-après.

TABLEAU VII

| Essai n° | Catalyseur | Conditions de prétraitement du catalyseur. Températures d'oxydation et de calcination en °C | Quantités de catalyseur | | Température à laquelle il y a 20% de conversion, en °C | Maximum d'isomérisation | | Température à laquelle il y a 20% de craquage, en °C |
|---|---|---|---|---|---|---|---|---|
| | | | Poids en mg | Volume en ml | | % | Température en °C | |
| 1 | C2 | 400 | 160 | 0,6 | 195 | 52 | 245 | 245 |
| 2 | C3 | 400 | 200 | 0,5 | 190 | 55 | 232 | 231 |
| 3 | C4 | 400 | 130 | 0,6 | 197 | 65 | 249 | 269 |
| 4 | C7 | 400 | 85 | 0,5 | 200 | 55 | 252 | 230 |
| 5 | C8 | 400 | 125 | 0,6 | 205 | 55 | 255 | 275 |
| 6 | C9 | 400 | 200 | 0,5 | 231 | 50 | 281 | 330 |
| 7 | C10 | 400 | 190 | 0,5 | 208 | 55 | 262 | 272 |
| 8 | C11 | 400 | 186 | 0,6 | 192 | 58 | 227 | 227 |
| 9 | C13 | 300 | 420 | 0,6 | 167 | 50 | 190 | 190 |
| 10 | C14 | 300 | 420 | 0,6 | 147 | 55 | 172 | 172 |
| 11 | C14 | 420 | 390 | 0,6 | 180 | 55 | 220 | 220 |

Ce tableau permet de constater qu'avec les argiles pontées préparées par le procédé selon l'invention, on peut obtenir des catalyseurs d'isomérisation et de craquage efficaces à des températures relativement basses.

Avec une argile non pontée et contenant 1 % en poids de platine, ces réactions ne se produisent pas.

EXEMPLE 6

Cet exemple concerne l'isomérisation d'une charge de butène-1 à l'aide des argiles A1, A3, A5 et A7.

On a utilisé, pour effectuer ces essais, le dispositif schématisé sur la figure 3.

Dans un réacteur 1 de 25 ml, on place 140 milligrammes d'argile. Ce réacteur est relié par la ligne 2 à la vanne à trois voies 3, elle-même reliée par la ligne 4 à une pompe de circulation 5, elle-même reliée au réacteur 1 par la ligne 6.

Le réacteur 1 et les lignes 2, 4 et 6 forment ainsi une boucle, dont le volume total est de 1,3 l et dans laquelle on peut :

- faire le vide à l'aide de la ligne 7, de la vanne à trois voies 8 et de la ligne 9 reliée à une pompe à vide non représentée,
- prélever des échantillons de gaz pour analyse par la ligne 7, la vanne à trois voies 8 et la ligne 10 reliée à un chromatographe non représenté.
- introduire un gaz comme le butène 1 par la ligne 11, munie d'une vanne 12 et reliée à la ligne 2.

Le réacteur est muni d'un système de chauffage non représenté.

Les essais se déroulent de la façon suivante : après avoir placé l'argile dans le réacteur, on dégaze la boucle à l'aide de la pompe à vide 5, la température du réacteur étant de 250°C. Quand la pression est de $10^{-6}$ mm de mercure, on isole le circuit de vide et on abaisse la température du réacteur à 100°C.

On introduit alors dans la boucle du butène-1 en quantité telle que la pression soit de 40 mm de mercure.

On met en fonctionnement la pompe de circulation 5 et on prélève périodiquement des échantillons, que l'on analyse par chromatographie.

Les résultats obtenus sont représentés sur la figure 4.

On constate, d'après cette figure 4, que les argiles préparées par le procédé selon l'invention ont une très bonne activité isomérisante.

## REVENDICATIONS

1.- Procédé de préparation d'argiles pontées, caractérisé en ce qu'on effectue la dialyse d'un mélange d'une solution aqueuse d'au moins un hydroxyde d'un métal et d'une suspension aqueuse d'argile.

2.- Procédé selon la revendication 1, caractérisé en ce que l'hydroxyde est choisi dans le groupe formé par les hydroxydes des éléments des groupes IIA, IIIA, IVA, VA, VIA, VIIA, VIII, IB, IIB, IIIB, IVB, VB et VIB de la classification périodique des éléments.

3.- Procédé selon l'une des revendications 1 et 2, caractérisé en ce que l'argile est choisie dans le groupe formé par des argiles gonflantes (smectites), naturelles ou synthétiques.

4.- Procédé selon l'une des revendications 1 à 3, caractérisé en ce que la concentration de l'ion du métal de l'hydroxyde, dans le mélange de la suspension d'argile et de la solution d'hydroxyde, exprimée en milliéquivalent par gramme d'argile, est comprise entre 6 et 60 et, de préférence, entre 10 et 30.

5.- Procédé selon l'une des revendications 1 à 4, caractérisé en ce que la concentration de l'argile, dans le mélange de la suspension d'argile et de la solution d'hydroxyde, est comprise entre 0,1 et 4 % en poids et, de préférence, comprise entre 0,8 et 1,5 % en poids.

6.- Procédé selon l'une des revendications 1 à 5, caractérisé en ce que le degré d'oxydation du métal M de l'hydroxyde est égal à I et en ce que le rapport $\frac{OH^-}{M^I}$ est compris entre 0,2 et 1, de préférence entre 0,2 et 0,8 et, mieux encore, entre 0,3 et 0,7.

7.- Procédé selon l'une des revendications 1 à 5, caractérisé en ce que le degré d'oxydation du métal M de l'hydroxyde est égal à II et en ce que le rapport $\frac{OH^-}{M^{II}}$ est compris entre 0,2 et 2, de préférence entre 0,4 et 1,8 et, mieux encore, entre 0,5 et 1,4.

8.- Procédé selon l'une des revendications 1 à 5, caractérisé en ce que le degré d'oxydation du métal M de l'hydroxyde est égal à III et en ce que le rapport $\frac{OH^-}{M^{III}}$ est compris entre 0,3 et 3, de préférence entre 0,6 et 2,4 et, mieux encore, entre 0,8 et 1,8.

9. - Procédé selon l'une des revendications 1 à 5, caractérisé en ce que le degré d'oxydation du métal M de l'hydroxyde est égal à IV et en ce que le rapport $\frac{OH^-}{M^{IV}}$ est compris entre 0,4 et 4, de préférence entre 0,8 et 3,6 et, mieux encore, entre 1,0 et 2,8.

10.- Procédé selon la revendication 8, caractérisé en ce que le métal de l'hydroxyde est au moins un métal choisi dans le groupe constitué par l'aluminium, le chrome et le holmium.

11.- Procédé selon la revendication 9, caractérisé en ce que le métal de l'hydroxyde est au moins un métal choisi dans le groupe constitué par le zirconium et le titane.

12.- Procédé selon l'une des revendications 1 à 11, caractérisé en ce que la dialyse est effectuée à l'aide d'une membrane semi-perméable choisie dans le groupe constitué par les membranes organiques.

13.- Procédé selon la revendication 12, caractérisé en ce que la membrane est choisie dans le groupe constitué par les membranes à base de cellulose régénérée.

14.- Les argiles préparées par un procédé selon l'une des revendications 1 à 13.

15.- Les catalyseurs comprenant une argile selon la revendication 14.

16.- Les catalyseurs constitués par une argile selon la revendication 15.

17.- Les catalyseurs selon la revendication 16, comprenant en outre au moins un des éléments des groupes IA, IIA, IIIA, IVA, VA, VIA, VIIA, VIII, IB, IIB, IIIB, IVB, VB et VIIB de la classification périodique des éléments.

18.- Un catalyseur selon la revendication 17, caractérisé en ce que l'élément qu'il comprend est un élément du groupe VIII.

19.- Un catalyseur selon la revendication 18, caractérisé en ce que l'élément du groupe VIII est le platine.

20.- Application des argiles et des catalyseurs selon l'une des revendications 14 à 19 à la conversion d'hydrocarbures paraffiniques ou oléfiniques.

21.- Application selon la revendication 20, caractérisée en ce que la réaction de conversion des hydrocarbures est une réaction d'isomérisation ou de craquage, éventuellement en présence d'hydrogène.

22.- Application selon la revendication 21, caractérisée en ce que les réactions de craquage et d'isomérisation s'effectuent en présence d'hydrogène et à l'aide d'un catalyseur constitué par un catalyseur contenant du platine déposé sur une argile selon la revendication 14.

0073718

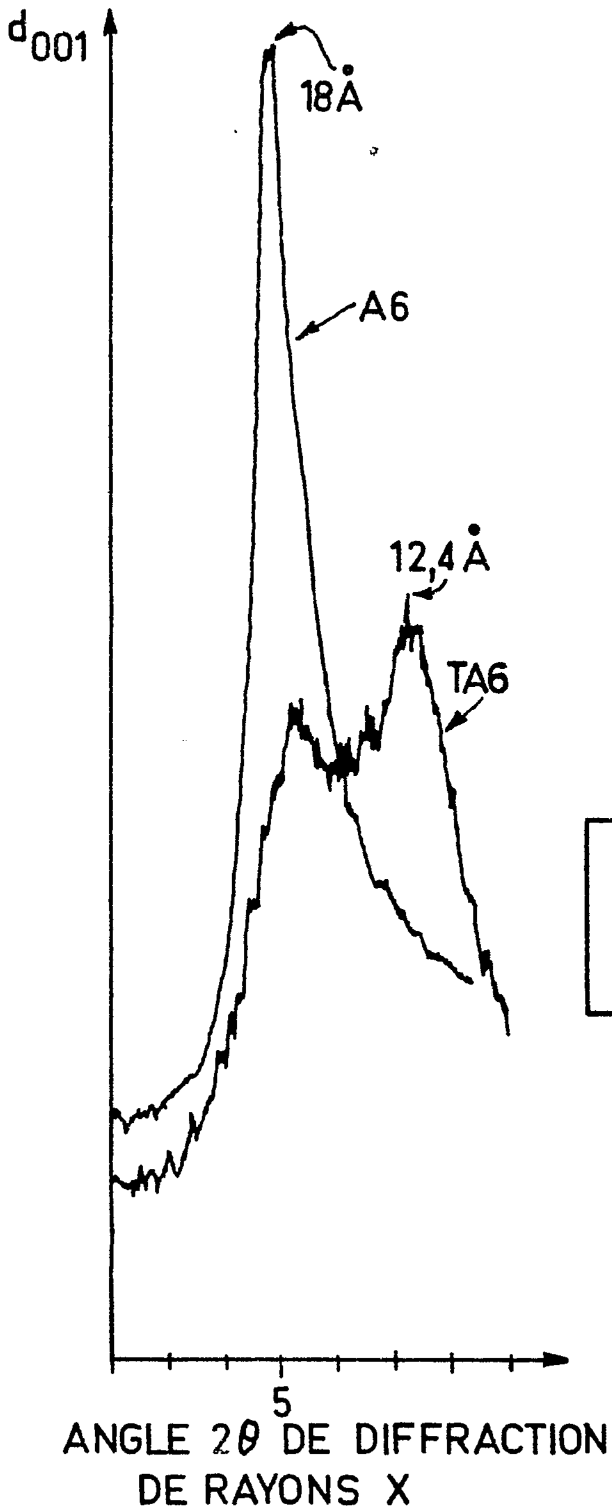


FIG.1

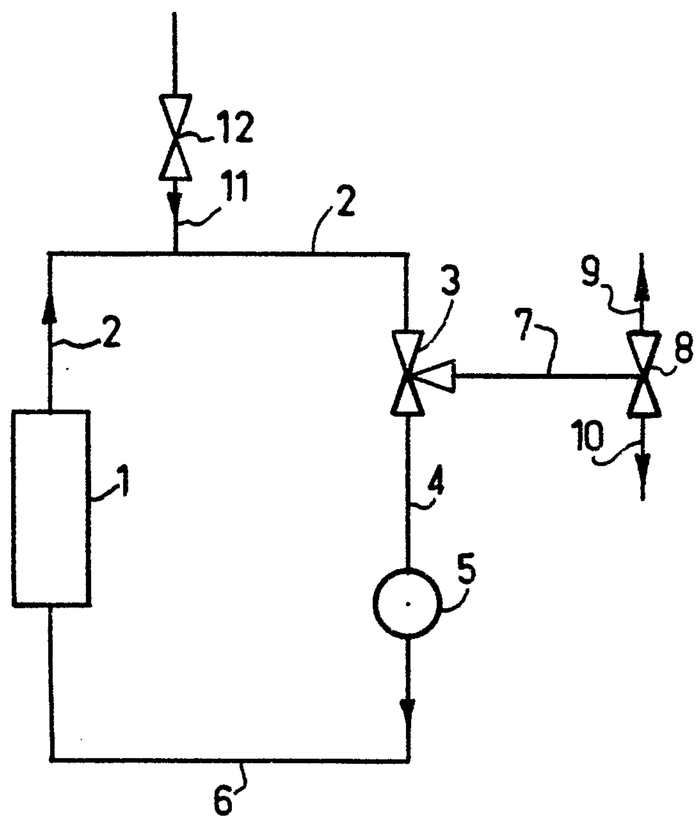


FIG.3

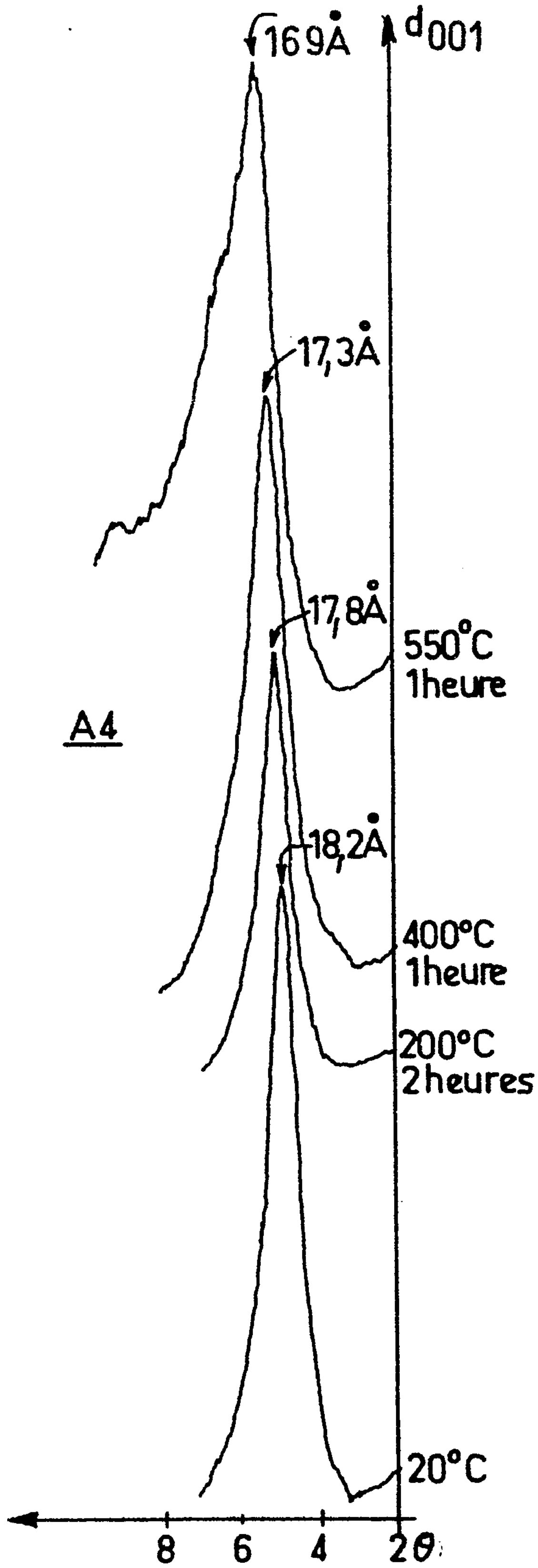
16,9Å
d001
17,3Å
17,8Å
550°C
1heure
A4
18,2Å
400°C
1heure
200°C
2heures
20°C
8
6
4
2θ

FIG.2a

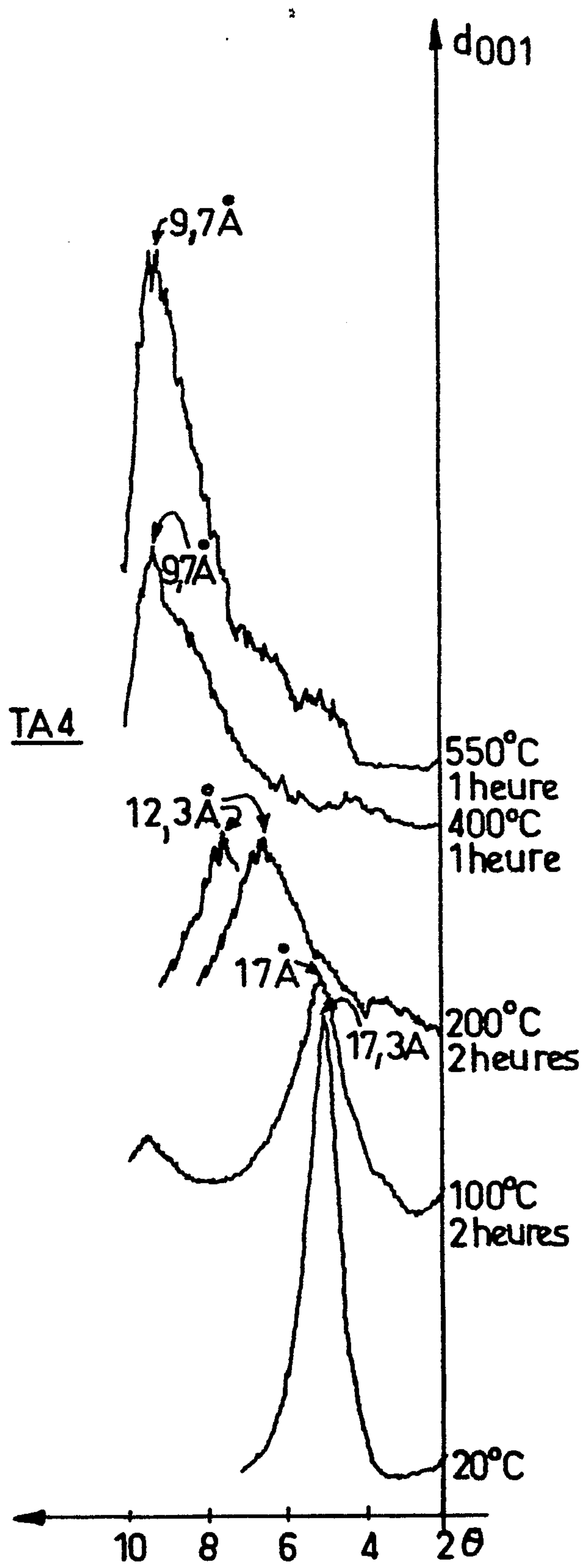
d001
9,7Å
9,7Å
TA4
550°C
1heure
400°C
1heure
12,3Å ?
17Å
17,3Å
200°C
2heures
100°C
2heures
20°C
10
8
6
4
2θ

FIG.2b

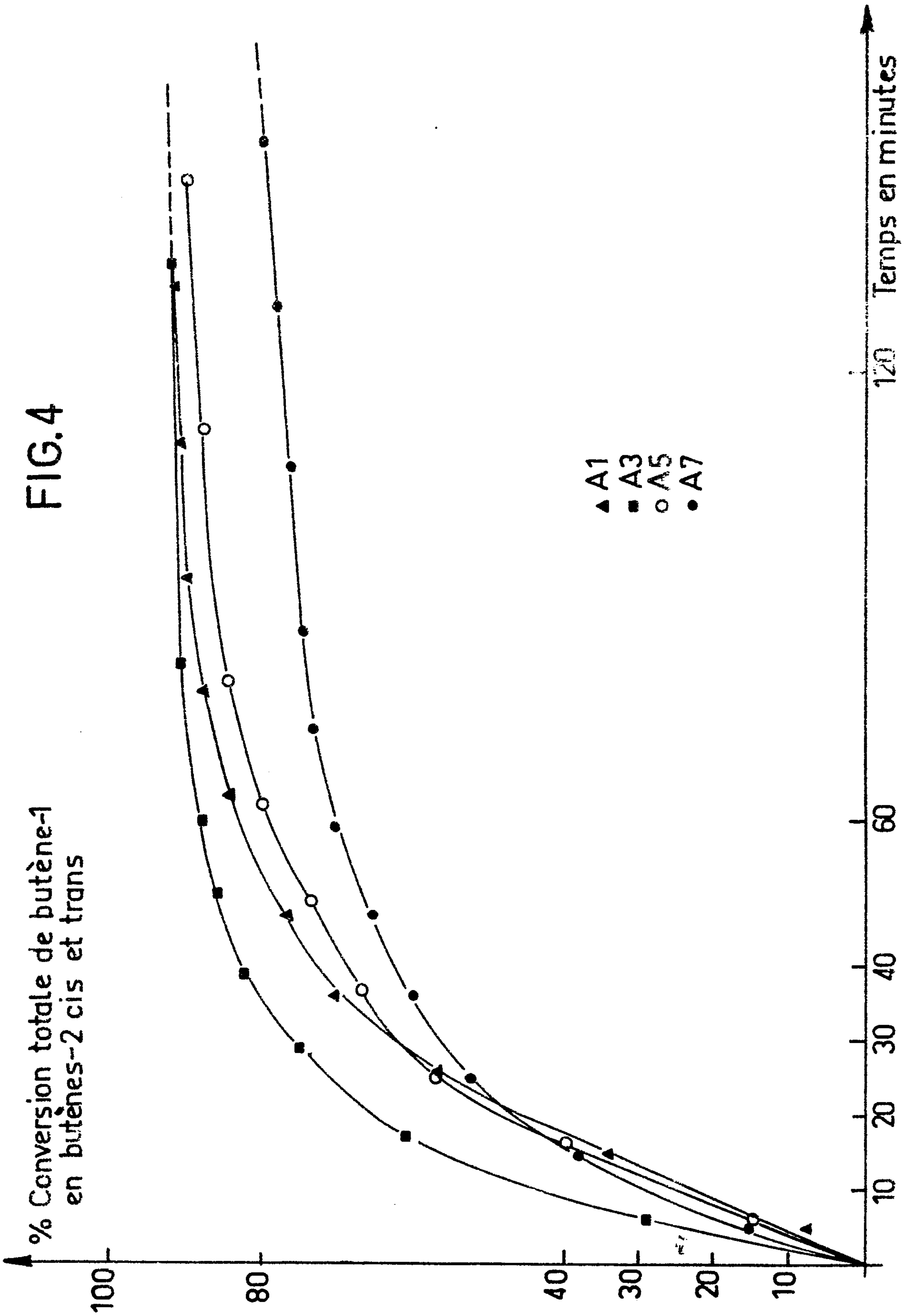
% Conversion totale de butène-1
en butènes-2 cis et trans
100
80
40
30
20
10
10
20
30
40
60
120
Temps en minutes
▲ A1
■ A3
○ A5
● A7

FIG.4

Office européen des brevets

# RAPPORT DE RECHERCHE EUROPEENNE

0073718

Numéro de la demande

EP 82 40 1585

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl. 3) |
| A | US-A-4 216 188 (J.SHABRIA)<br>*Revendications 1-15; colonne 4, lignes 34-68; colonne 5, lignes 1-20* | 1-5,8, 10,15-22 | B 01 J 21/16<br>C 01 B 33/26<br>B 01 J 29/02 |
| A | US-A-4 060 480 (M.G.REED)<br>*Revendications 1-12; colonne 1, lignes 40-68* | 1-5,8, 10,15-22 | |
| A | US-A-4 248 739 (D.E.W.VAUGHAM)<br>*Revendications 1-10* | 1-3,10,11,15-22 | |
| | | | DOMAINES TECHNIQUES RECHERCHES (Int. Cl. 3) |
| D,A | FR-A-2 394 324 (W.R.GRACE & CO)<br>*Revendications 1-16; page 8, lignes 35-37; page 9, lignes 1-5* | | B 01 J 21/00<br>B 01 J 29/00<br>C 01 B 33/00<br>B 01 D 13/00 |

Le présent rapport de recherche a été établi pour toutes les revendications

| Lieu de la recherche | Date d'achèvement de la recherche | Examinateur |
|---|---|---|
| LA HAYE | 06-12-1982 | SCHURMANS H.D.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82